# EUROPÄISCHE PATENTSCHRIFT

(11) **EP 0 732 324 B1**
(45) Veröffentlichungstag und Bekanntmachung des Hinweises auf die Patenterteilung: **13.10.1999**
(21) Anmeldenummer: 96103640.7
(22) Anmeldetag: 08.03.1996
(51) Int. Cl.: C07D 207/26, C07D 211/76, C07D 223/10

(54) **Verfahren zur Herstellung von N-Vinyllactamen**
Process for the preparation of N-vinyllactams
Procédé pour la préparation de N-vinyllactames

(30) Priorität: 15.03.1995 DE 19509362
(43) Veröffentlichungstag der Anmeldung: 18.09.1996
(73) Patentinhaber: BASF AKTIENGESELLSCHAFT, 67056 Ludwigshafen (DE)
(72) Erfinder: Schmidt-Radde, Martin, Dr., 67259 Beindersheim (DE); Heider, Marc, Dr., 67433 Neustadt (DE); Dams, Albrecht, Dr., 67157 Wachenheim (DE); Rust, Harald, 37435 Neustadt (DE)

(56) Entgegenhaltungen:
- WO-A-89/09210
- DE-A- 3 215 093
- GB-A- 799 924
- GB-A- 1 045 627
- NL-C- 95 983

## Beschreibung

Die vorliegende Erfindung betrifft ein Verfahren zur Herstellung von N-Vinyllactamen durch destillative Umsetzung von Lactamen mit einer wäßrigen Alkalihydroxid-Lösungen und anschließende Umsetzung mit Acetylen.

Aus Liebigs Ann. Chem. 601, 128 bis 138 (1956) ist die Umsetzung von Lactamen und Amiden mit Acetylen in Gegenwart von katalytischen Mengen von stark basischen Alkalimetallen bekannt. Durch die Verwendung von elementarem Alkalimetall werden Wasserspuren im Ansatz vermieden. Diese Methode hat wegen der Schwierigkeiten im Umgang mit Alkalimetallen technisch keine Relevanz.

In WO-A-89/09210 wird die Verwendung von Alkalimetallsalzen sterisch gehinderter Alkohole empfohlen, wodurch die Entfernung des entstehenden sterisch gehinderten Alkohols entfallen kann, da dieser eine Ringöffnung des Lactams nicht initiieren. In der Praxis hat dieses Verfahren aber den Nachteil, daß pulver-förmigen, stark basische Verbindungen gehandhabt werden müssen. Diese Katalysatoren sind hochpreisig. Ferner liegen die entstehenden Alkohole auch in den Austrägen der Lactam-Vinylierung vor und müssen anschließend abgetrennt und entsorgt werden.

In US-A-31 86 706 wird die Herstellung von Alkalisalzen von Pyrrolidon für die Herstellung von Polymeren durch Reaktion von Alkalimetallsalzen in Lösungsmitteln wie Alkoholen, Ethern oder Kohlenwasserstoffen mit Pyrrolidon im Vakuum bei Temperaturen von 170 bis 250°C beschrieben.

Aus Ind. Eng. Chem. Proc. Design. & Dev. 1 (1962) 137 bis 141 ist bekannt, daß Kaliumhydroxid als Katalysator für die Vinylierung von Lactamen aufgrund rascher Hydrolyse nur eine kurze Lebenszeit hat. Durch Wasser wird die Hydrolyse stark beschleunigt. Aus diesem Grund wird hier Kaliumhydroxid erst im Reaktor dem Lactam zugeführt, was aber apparativ sehr aufwendig ist und nur geringe Verweilzeiten des Lactams im Reaktor ermöglicht.

In DE-A-32 15 093 wird festes Kaliumhydroxid mit Pyrrolidon zur Herstellung von Vinylierungsansätzen verwendet. Zum Erhalt guter Ergebnisse muß eine vollständige Entfernung des Wassers erfolgen. Das dort beschriebene Verfahren ist diskontinuierlich, und der Katalysator wird in fester Form verwendet, was Schwierigkeiten bei der Dosierung und bei der Handhabung mit sich bringt.

Der vorliegenden Erfindung lag daher die Aufgabe zugrunde, den zuvor genannten Nachteilen abzuhelfen.

Demgemäß wurde ein neues und verbessertes Verfahren zur Herstellung von N-Vinyllactamen der allgemeinen Formel I 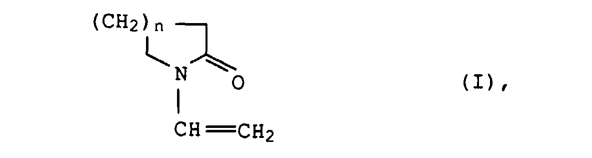 in der n für 1 bis 3 steht, gefunden, welches dadurch gekennzeichnet ist, daß man Lactame der allgemeinen Formel II 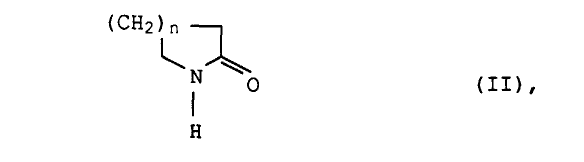 in der n für 1 bis 3 steht, mit 10 bis 90 gewichtsprozentigen wäßrigen Alkalihydroxid-Lösungen destillativ bei Temperaturen von 50 bis 250°C, Drücken von 1 bis 100 mbar und einer Verweilzeit von 0,1 bis 5 Stunden und anschließend bei Temperaturen von 60 bis 250°C und Drücken von 1 bis 100 bar mit Acetylen umsetzt.

Das erfindungsgemäße Verfahren kann wie folgt durchgeführt werden:

Die wässrige Alkalihydroxid-Lösung und das Lactam II können im oberen Drittel, bevorzugt im oberen Viertel, besonders bevorzugt am Kopf einer Kolonne gemeinsam oder bevorzugt in getrennten Zuläufen eingespeist werden. Der Druck in der Kolonne beträgt in der Regel 1 bis 100 mbar, bevorzugt 1 bis 30 mbar, besonders bevorzugt 2 bis 20 mbar. Als Kolonnen können sowohl Füllkörper- als auch Bodenkolonnen verwendet werden. In der Regel haben diese Kolonnen mindestens 2 theoretische Böden, also 2 bis 100 theoretische Böden, bevorzugt 2 bis 10 theoretische Böden, besonders bevorzugt 3 bis 6 theoretische Böden. Die Kolonne kann bei Temperaturen von 50 bis 250°C, bevorzugt 70 bis 180°C betrieben werden. Die Verweilzeit beträgt in der Regel 0,1 bis 5 Stunden, bevorzugt 0,5 bis 2 Stunden, besonders bevorzugt 0,8 bis 1,5 Stunden.

Als Alkalihydroxid-Lösungen eignen sich solche von Lithiumhydroxid, Natriumhydroxid, Kaliumhydroxid, Rubidiumhydroxid und Caesiumhydroxid, bevorzugt von Lithiumhydroxid, Natriumhydroxid und Kaliumhydroxid, besonders bevorzugt von Kaliumhydroxid.

Der Gehalt des Alkalihydroxids in der wässrigen Lösung beträgt in der Regel 10 bis 90 Gew.-%, bevorzugt 30 bis 60 Gew.-%, besonders bevorzugt 45 bis 55 Gew.-%, insbesondere 50 Gew.-%.

Während das Wasser in der Regel über den Kopf der Kolonne entfernt wird, fällt der Vinylierungsansatz im Sumpf an und wird mit Acetylen in der Regel in einem getrennten Reaktionsgefäß bei Temperaturen von 60 bis 250°C, Drücken von 1 bis 100 bar umgesetzt.

Diese Verfahrensweise ist für Pyrrolidon, Piperidon und Caprolactam gleichermaßen geeignet.

### Beispiele

### A) Herstellung der Vinylierungsansätze

### Beispiele 1 bis 18

In eine Packungskolonne (Nennweite 43 mm) mit ca. 3 theoretischen Trennstufen wurden pro Stunde 500 g Caprolactam und 20 g 50 % KOH in Wasser gepumpt. Druck und Temperaturen sind der Tabelle zu entnehmen. Im Sumpf fielen 506 g/h Caprolactam-Ansatz an, im Destillat erhielt man 14 g/h Wasser.

| | | Temperaturen | | | Bestimmungen | | |
|---|---|---|---|---|---|---|---|
| Beisp.-Nr. | Druck [mbar] | Heizöl [°C] | Sumpf [°C] | Kopf [°C] | KOH [%] | H₂O [%] | Akt.Kat. [%] |
| 1 | 5 | 127 | 113 | 82 | 1,97 | 0,08 | 76 |
| 2 | 5 | 127 | 113 | 83 | 1,72 | 0,09 | 100 |
| 3 | 5 | 127 | 113 | 83 | 1,89 | 0,09 | 78 |
| 4 | 5 | 127 | 113 | 84 | 1,85 | 0,05 | 78 |
| 5 | 5 | 127 | 113 | 83 | 1,88 | 0,05 | 80 |
| 6 | 5 | 127 | 113 | 83 | 2,04 | 0,1 | 76 |
| 7 | 5 | 127 | 109 | 90 | 1,6 | 0,07 | 67 |
| 8 | 5 | 127 | 112 | 80 | 2,36 | 0,1 | 76 |
| 9 | 5 | 127 | 111 | 81 | 1,81 | 0,08 | 86 |
| 10 | 5 | 127 | 111 | 82 | 1,55 | 0,07 | 83 |
| 11 | 5 | 127 | 112 | 78 | 1,73 | 0,09 | 78 |
| 12 | 5 | 127 | 113 | 87 | 2 | 0,1 | 81 |
| 13 | 10 | 140 | 119 | 82 | 2,04 | 0,13 | 82 |
| 14 | 10 | 140 | 121 | 76 | 2 | 0,08 | 80 |
| 15 | 10 | 140 | 121 | 77 | 2,1 | 0,08 | 78 |
| 16 | 30 | 160 | 141 | 68 | 1,97 | 0,03 | 62,8 |
| 17 | 30 | 160 | 141 | 73 | 1,58 | 0,01 | 74,2 |
| 18 | 30 | 160 | 141 | 77 | 1,77 | 0,01 | 70,6 |

### B) Umsetzung der Ansätze mit Acetylen

### Beispiele I und II

In einen 300-ml-Hochdruckautoklaven wurden 150 g eines unter A) hergestellten Ansatzes eingefüllt, der Autoklav mit 2 bar Stickstoff inertisiert und auf 90°C aufgeheizt. Anschließend wurde Acetylen auf einen Gesamtdruck von 20 bar nachgepresst. Verbrauchtes Acetylen wurde durch kontinuierliches Nachpressen ersetzt. Nach beendeter Reaktion wurde abgekühlt, entspannt und der Reaktionsaustrag analysiert.

| Beisp.-Nr. | KOH [Gew.-%] | Wasser [Gew.-%] | Akt. Kat. [%] | Laufzeit [h] | Acetylenmenge [l] | Umsatz [%] | Selektivität [%] |
|---|---|---|---|---|---|---|---|
| I | 1,79 | 0,1 | 86 | 9,5 | 27,2 | 93,1 | 99 |
| II | 1,88 | 0,05 | 80 | 11,5 | 25,7 | 91,8 | 100 |

## Patentansprüche

1. Verfahren zur Herstellung von N-Vinyllactamen der allgemeinen Formel I 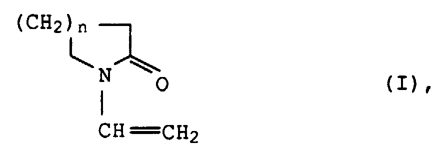 in der n für 1 bis 3 steht, dadurch gekennzeichnet, daß man Lactame der allgemeinen Formel II 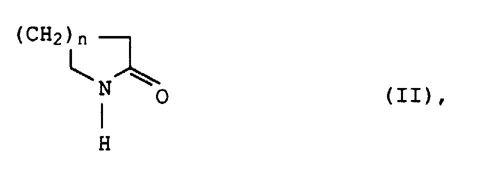 in der n für 1 bis 3 steht, mit 10 bis 90 gewichtsprozentigen wäßrigen Alkalihydroxid-Lösungen destillativ bei Temperaturen von 50 bis 250°C, Drücken von 1 bis 100 mbar und einer Verweilzeit von 0,1 bis 5 Stunden und anschließend bei Temperaturen von 60 bis 250°C und Drücken von 1 bis 100 bar mit Acetylen umsetzt.

2. Verfahren zur Herstellung von N-Vinyllactamen nach Anspruch 1, dadurch gekennzeichnet, daß man die destillative Umsetzung in einer Kolonne mit mindestens 2 theoretischen Böden durchführt.

3. Verfahren zur Herstellung von N-Vinyllactamen nach Anspruch 1 und 2, dadurch gekennzeichnet, daß man das Lactam II und die wäßrige Alkalihydroxid-Lösung bei der destillativen Umsetzung im oberen Drittel der Kolonne einspeist.

4. Verfahren zur Herstellung von N-Vinyllactamen nach den Ansprüchen 1 bis 3, dadurch gekennzeichnet, daß man die destillative Umsetzung bei Temperaturen von 70 bis 180°C durchführt.

5. Verfahren zur Herstellung von N-Vinyllactamen nach den Ansprüchen 1 bis 4, dadurch gekennzeichnet, daß man die destillative Umsetzung bei Drücken von 1 bis 30 mbar durchführt.

6. Verfahren zur Herstellung von N-Vinyllactamen nach den Ansprüchen 1 bis 5, dadurch gekennzeichnet, daß man die destillative Umsetzung mit einer Verweilzeit von 0,5 bis 2 Stunden durchführt.

7. Verfahren zur Herstellung von N-Vinyllactamen nach den Ansprüchen 1 bis 6, dadurch gekennzeichnet, daß man die Umsetzung mit 30 bis 60 gewichtsprozentigen wäßrigen Alkalihydroxid-Lösungen durchführt.

8. Verfahren zur Herstellung von N-Vinyllactamen nach den Ansprüchen 1 bis 6, dadurch gekennzeichnet, daß man die Umsetzung mit 45 bis 55 gewichtsprozentigen wäßrigen Alkalihydroxid-Lösungen durchführt.

9. Verfahren zur Herstellung von N-Vinyllactamen nach den Ansprüchen 1 bis 8, dadurch gekennzeichnet, daß man als wäßrige Alkalihydroxid-Lösungen wäßrige Kaliumhydroxid-Lösungen einsetzt.

## Claims

1. A process for the preparation of an N-vinyllactam of the formula I 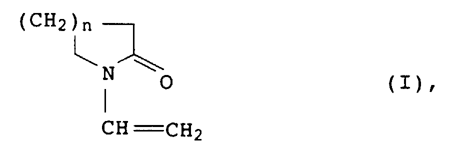 where n is from 1 to 3, which comprises reaction of a lactam of the formula II 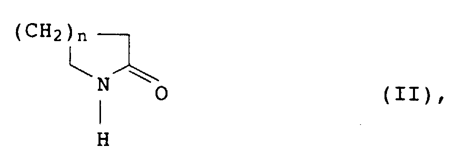 where n is from 1 to 3, with a from 10 to 90 percent strength by weight aqueous alkali metal hydroxide solution with distillation at from 50 to 250°C and from 1 to 100 mbar and with a residence time of from 0.1 to 5 hours and subsequent reaction with acetylene at from 60 to 250°C and from 1 to 100 bar.

2. A process for the preparation of an N-vinyllactam as claimed in claim 1, wherein the distillative reaction is carried out in a column having at least 2 theoretical plates.

3. A process for the preparation of an N-vinyllactam as claimed in claims 1 and 2, wherein the lactam II and the aqueous alkali metal hydroxide solution are fed into the upper third of the column during the distillative reaction.

4. A process for the preparation of an N-vinyllactam as claimed in any of claims 1 to 3, wherein the distillative reaction is carried out at from 70 to 180°C.

5. A process for the preparation of an N-vinyllactam as claimed in any of claims 1 to 4, wherein the distillative reaction is carried out at from 1 to 30 mbar.

6. A process for the preparation of an N-vinyllactam as claimed in any of claims 1 to 5, wherein the distillative reaction is carried out with a residence time of from 0.5 to 2 hours.

7. A process for the preparation of an N-vinyllactam as claimed in any of claims 1 to 6, wherein the reaction is carried out using a from 30 to 60 percent strength by weight aqueous alkali metal hydroxide solution.

8. A process for the preparation of an N-vinyllactam as claimed in any of claims 1 to 6, wherein the reaction is carried out using a from 45 to 55 percent strength by weight aqueous alkali metal hydroxide solution.

9. A process for the preparation of an N-vinyllactam as claimed in any of claims 1 to 8, wherein the aqueous alkali metal hydroxide solution used is aqueous potassium hydroxide solution.

## Revendications

1. Procédé de préparation de N-vinyl-lactames de formule générale I 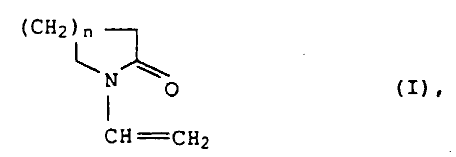 dans laquelle n vaut de 1 à 3, caractérisé en ce que l'on fait réagir par distillation des lactames de formule générale II 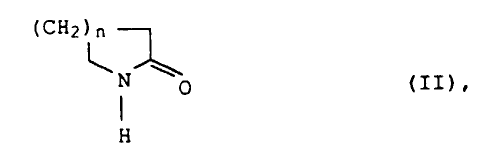 dans laquelle n vaut de 1 à 3, avec des solutions aqueuses d'hydroxydes de métaux alcalins à 10-90 pourcent en poids, à des températures de 50-250°C, sous des pressions de 1-100 mbar et pour une durée de séjour de 0,1-5 heures puis avec de l'acétylène à des températures de 60-250°C et sous des pressions de 1-100 bar.

2. Procédé de préparation de N-vinyl-lactames selon la revendication 1, caractérisé en ce que l'on mène la réaction par distillation dans une colonne ayant au moins deux plateaux théoriques.

3. Procédé de préparation de N-vinyl-lactames selon la revendication 1 ou 2, caractérisé en ce que, lors de la réaction par distillation, on introduit le lactame II et la solution aqueuse d'hydroxyde de métal alcalin dans le tiers supérieur de la colonne.

4. Procédé de préparation de N-vinyl-lactames selon l'une quelconque des revendications 1 à 3, caractérisé en ce que l'on mène la réaction par distillation à des températures de 70-180°C.

5. Procédé de préparation de N-vinyl-lactames selon l'une quelconque des revendications 1 à 4, caractérisé en ce que l'on mène la réaction par distillation sous des pressions de 1-30 mbar.

6. Procédé de préparation de N-vinyl-lactames selon l'une quelconque des revendications 1 à 5, caractérisé en ce que l'on mène la réaction par distillation avec un temps de séjour de 0,5-2 heures.

7. Procédé de préparation de N-vinyl-lactames selon l'une quelconque des revendications 1 à 6, caractérisé en ce que l'on mène la réaction avec des solutions aqueuses d'hydroxydes de métaux alcalins à 30-60 pourcent en poids.

8. Procédé de préparation de N-vinyl-lactames selon l'une quelconque des revendications 1 à 6, caractérisé en ce que l'on mène la réaction avec des solutions aqueuses d'hydroxydes de métaux alcalins à 45-55 pourcent en poids.

9. Procédé de préparation de N-vinyl-lactames selon l'une quelconque des revendications 1 à 8, caractérisé en ce que l'on utilise en tant que solutions aqueuses d'hydroxydes de métaux alcalins des solutions aqueuses d'hydroxyde de potassium.
